(19) Europäisches Patentamt
European Patent Office
Office européen des brevets

(11) Publication number: **0 000 539**
**A1**

(12) # EUROPEAN PATENT APPLICATION

(21) Application number: 78100432.0

(22) Date of filing: 19.07.78

(51) Int. Cl.[2]: C 07 F 1/00,A 01 N 11/04

(30) Priority: 27.07.77 CH 9283/77

(43) Date of publication of application:
07.02.79 Bulletin 79/3

(84) Designated contracting states:
BE CH DE FR GB NL

(71) Applicant: CIBA-GEIGY AG
Patentabteilung Postfach
CH-4002 Basel. (CH)

(72) Inventor: Eberle, Jürg
Vorderbergrain 7
CH-4104 Oberwil. (CH)

(72) Erfinder: de Wijs, Jean Jacques
Waldhofstrasse 26
CH-4310 Rheinfelden. (CH)

(72) Erfinder: Kunz, Walter, Dr.
Im Goldbrunnen 55
CH-4104 Oberwil. (CH)

(72) Erfinder: Hubele, Adolf, Dr.
Obere Egg 9
CH-4465 Magden. (CH)

(72) Erfinder: Eckhardt, Wolfgang, Dr.
Breslauer Strasse 16
D-7850 Lörrach. (DE)

(54) **Copper complexes of N-pyrazole, N-imidazole and N-triazole acetanilides, their preparation and their use as fungicides.**

(57) Copper complexes of N-pyrazole-, N-imidazole- and N-triazole-acetanilide derivatives of formula:

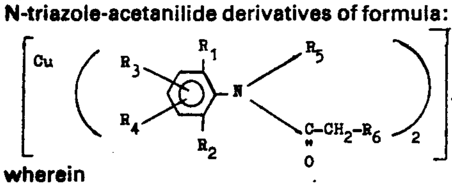 An . $pH_2O$ (I)

wherein
$R_1$ and $R_2$ represent independently of each other methyl, ethyl, methoxy, ethoxy or halogen,
$R_3$ represents hydrogen, $C_1$-$C_3$-alkyl, $C_1$-$C_4$-alkoxy or halogen,
$R_4$ represents hydrogen or methyl
$R_5$ represents 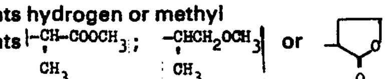 or
$R_6$ represents 1,2-pyrazole, 1,3-imidazole, 1,2,4-triazole (1) or 1,2,4-triazole (4)
A represents the anion of an organic or inorganic acid and
n stands for the number 1 or 2
p stands for a number from 1 to 12 inclusive, their preparation and use as fungicides.

EP 0 000 539 A1

5-11276/-

TITLE MODIFIED
see front page

Copper complexes of N-pyrazole-, N-imidazole-and N-triazole-acetanilide derivatives as fungicides.

The present invention relates to copper complexes of N-pyrazole-, N-imidazole-and N-triazole-aniline derivatives, a process for their preparation, compositions containing them and their use in combatting phytopathogenic fungi.

The aniline derivatives which form the basis of the copper complexes of the invention are in part known from German Offenlegungsschrift No. 2,643,477 which also describes their fungicidal activity.

The complexes of the present invention have been found to possess a high fungicidal activity over a prolonged period.

More particularly the present invention provides complexes of the formula I

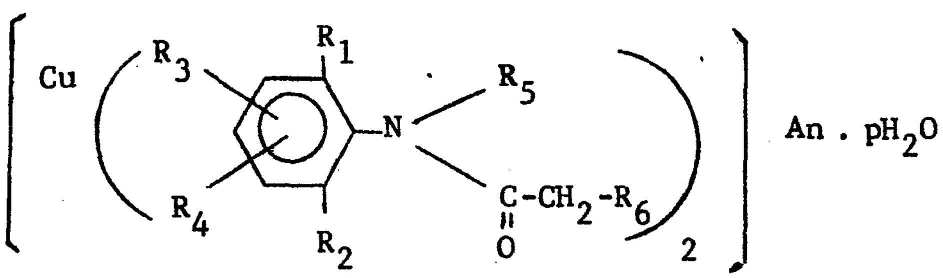

(I)

wherein

$R_1$ and $R_2$ represent independently of each other methyl, ethyl, methoxy, ethoxy or halogen,

$R_3$ represents hydrogen, $C_1$-$C_3$-alkyl, $C_1$-$C_4$-alkoxy or halogen,

$R_4$ represents hydrogen or methyl,

$R_5$ represents

$-CHCOOCH_3$ ; $-CHCH_2OCH_3$ or 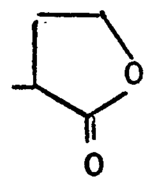

$\;\;|\qquad\qquad\qquad\;\;|$

$\;CH_3\qquad\qquad\quad CH_3$

$R_6$ represents 1,2-pyrazole, 1,3-imidazole, 1,2,4-triazole(1) or 1,2,4-triazole-(4),

A represents the anion of an organic or inorganic acid and

n stands for the number 1 or 2 and

p stands for a number from 1 to 12 inclusive.

According to the number of carbon atoms present the following are to be understood as alkyl or as alkyl moiety of an alkoxy group: methyl, ethyl, n-propyl, iso-propyl, n-butyl, iso-butyl, sec- butyl or tert-butyl.

Halogen stands for fluorine, chlorine, bromine or iodine.

The following are examples of acids from which anion A may be derived e.g. hydrochloric acid, hydrobromic acid, hydriodic acid, nitric acid, phosphoric acid, sulphuric acid, methanesulphonic acid, acetic acid, haloacetic acids, propionic acid, halopropionic acids, butyric acid, lactic acid, oxalic acid, maleic acid or benzoic acid.

The number p indicates the degree of hydration and will not necessarily be a whole number.

The complexes of the invention are stoichiometrically precisely defined compounds.

The complexes of the formula I may be prepared analogously to known methods for example as follows

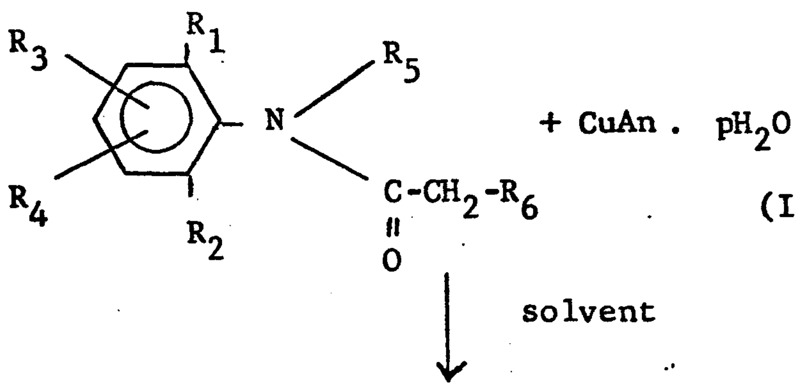


(III)

(I)

suitable solvents are for example alcohols such as methanol and ethanol; ketones such as acetone and methylethylketone; acetonitrile, dimethylformamide, dimethylsulphoxide, methylcellosolve and water as well as mixtures thereof. The process is advantageously carried out in the presence of ammonium chloride. The reaction temperature lies between 0° and 100° C preferably 20°-50° C. The process is carried out under normal pressure.
The above process also forms part of the invention.
The complexes of the formula I contain in the group $R_5$ an asymetrical carbon atom adjacent to the aniline nitrogen and may be obtained as optical antipodes in conventional

manner (e.g. employing already separated starting materials). The two configurations of such a compound of the formula I exhibit different degrees of microbicidal activity. The effect of further possible centres of asymetry and the atropisomerism about the phenyl — N < axis on the microbicidal action of the entire molecule is apparently negligible. Carrying out the process of the invention without attempting to separate the pure isomers of the complex produces a racemic mixture thereof.

In a further embodiment of the invention the complexes of the formula I are mixed with suitable carriers and/or other additives to give fungicidal compositions.

Suitable carriers and additives can be solid or liquid and are those normally used in the art of formulation, for example natural or regenerated mineral substances, solvents, dispersants, wetting agents, tackifiers, thickeners, binders or fertilisers. The preparation of the compositions is effected in known manner by intimately mixing the constituents.

The following are examples of compositions according to the invention (percentages refer to advantageous amounts by weight of active substance):

Solid formulations:

dusts, tracking agents, (up to 10 %), granules (coated granules, impregnated granules and homogenous granules); pellets (1 to 80 %);

Liquid formulations:

a) active substance concentrates which are dispersible in water: wettable powders, pastes; (25-90 % in

commercial packs, 0.01 to 15% in ready for use solutions); emulsions; concentrated solutions (10 to 50 %; 0.01 to 15 % in ready for use solutions).
b) Solutions, aerosols.

The content of active substance in the above described compositions is between 0.1 % and 90 % by weight.

The compounds of the formula I can be used together with other suitable pesticides, for example fungicides, insecticides, acaricides or active substances which influence plant growth, in order to broaden the activity spectrum of the formulations and to adapt them to prevailing circumstances.

The complexes of the formula I are suitable for the protection of horticultural and agricultural crops and cultures from attack by phytopathogenic fungi. Examples of such crops and cultures are maize, vegetables, sugarbeet, soya, ground nuts, fruit trees, ornamentals, vines, hops, cucumber plants (cucumber, marrows, melons, squash), solanaceae such as potatoes, tobacco plants and tomatoes and also banana, cocoa and rubber plants.

With the active compounds of the formula I it is possible to inhibit or destroy the fungi which occur in plants or parts of plants (fruit, blossoms, leaves, stems, tubers, roots) in these and related cultures and also to protect from attack by such fungi parts of the plants which grow later. Typical of such fungi are those which belong to the following classes: Ascomycetes (e.g. Erysiphaceae); Basidiomycetes, in particular rust fungi; fungi imperfecti (e.g. Cercospora); and especially Oomycetes belonging to the class of the Phycomycetes, such as Phytophthora,

Pythium or Plasmopara. In addition, the compounds of the formula I possess a systemic action. They can also be used as seed dressing agents for protecting seeds (fruit, tubers, grains) and plant cuttings from fungal infections and from phytopathogenic fungi which occur in the soil.

A further aspect of the invention therefore concerns the use of the complexes of the Formula I in combatting phytopathogenic fungi.

Preferred complexes are those of the formula I wherein

$R_1$ represents methyl
$R_2$ represent methyl or chlorine and
$R_3$ represents hydrogen, chlorine, bromine or methyl.

Preferred as substituent $R_6$ is the 1,2,4-triazole group.

The anions of inorganic acids are preferred as the group A. X is preferably the $-\underset{CH_3}{\underset{|}{CH}}-COOCH_3$ group or 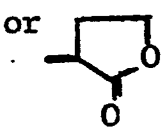 group

and especially the $-\underset{CH_3}{\underset{|}{CH}}-COOCH_3$ group.

Combination of the various compound groups are of particular interest.

The invention is illustrated by the following Examples. Unless otherwise stated, a compound of the formula I is always obtained as a racemic mixture of possible isomers.

Temperatures are given in degrees centigrade, pressures in millibars and parts and percentages are by weight.

A. Preparative examples

Example 1

Preparation of

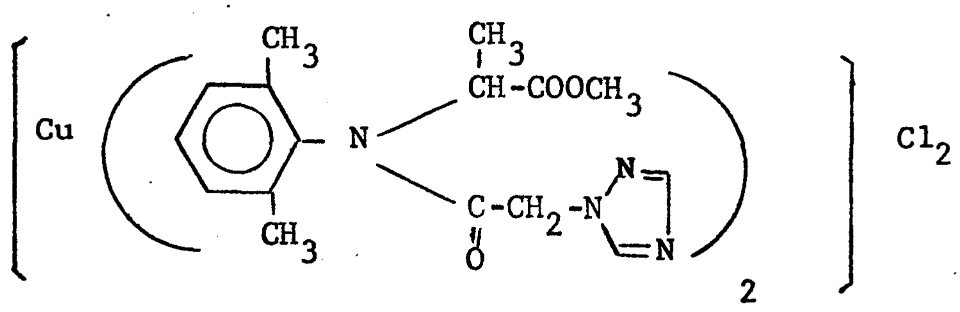

Copper complex of N-(1'-methoxycarbonyl-ethyl)-N-(1,2,4-triazol-1-yl)-acetyl-2,6-dimethylaniline (compound No. 2).

9.3 g of N-(1'-methoxycarbonyl-ethyl)-N-(1,2,4-triazol-1-yl)-acetyl-2,6-dimethylanilin were disolved in 75 ml of warm 95 % ethanol and reacted with a solution comprising 2.7 g of copper-(II)-chloride dihydrate in 10 ml. of water and 5 g of ammonium chloride in 20 ml of water. Precipition from the clear solution of the deep-blue crystals was initiated after a few minutes by scratching with a glass rod. After standing for 30 mins. at 30° the reaction mixture was cooled, filtered and washed through with cold water.

The copper complex thus obtained melted at 193-194°

Example 2

Preparation of

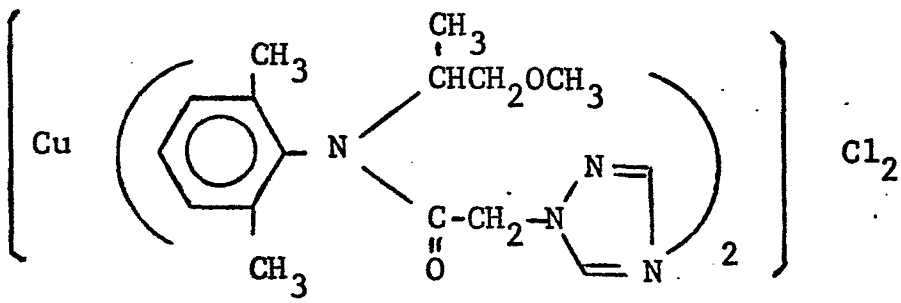

Copper complex of N-(1'-methyl-2'-methoxyethyl)-N-(1,2,4-triazol-1-yl)-acetyl-2,6-dimethyl anilin (compound no. 10)

9.1 g of N-(1'-methyl-2-methoxyethyl)-N-(1,2,4-triazol-1-yl)-acetyl-2,6-dimethyl aniline were disolved in 40 ml of warm 95 % ethanol and reacted with a solution comprising 2.7 g of copper-(II)-chloride dihydrate in 10 ml of water and 5 g of ammonium chloride in 20 ml of water. The rapidly formed blue suspension was diluted with 20 ml of water, stirred for a further 20 mins. and filtered. The blue crystals obtained were washed through with water and diethylether and dried, m.p. 138-139°.

Example 3

Preparation of

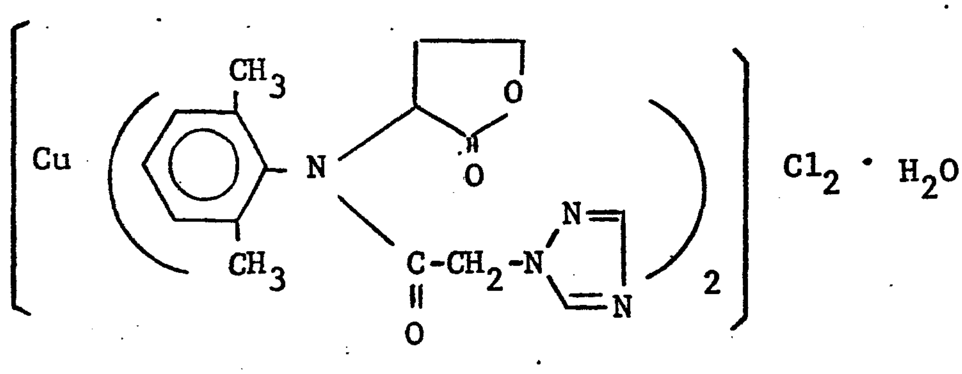

Copper complex of 3-(N-(1,2,4-triazol-1-yl-acetyl)-N-(2,6-dimethylphenyl)-amino-tetrahydro-2-furanone (compound no. 16).

3.2 g of 3-(N-(1,2,4-triazole-1-yl-acetyl)-N-(2,6-dimethylphenyl)-amino-tetrahydro-2-furanone were disolved at 50° in 22.5 ml of 95 % ethanol and reacted for 2 hours at 65° and 16 hours at room temperature with a solution comprising 1 g of copper-(II)-chloride dihydrate in 3 ml of water and 1.5 of ammonium chloride in 6ml of water.

The ice-cooled reaction mixture was then treated with 20 ml water, filtered, washed through with water and the obtained crystals dried under vacuum, m.p. 190-195°.

The following complexes of the formula I can be obtained analogously.

Table I $(R_5 = -\overset{CH_3}{|}{C}HCOOCH_3)$

| Cmpd. no. | $R_1$ | $R_2$ | $R_3$ | $R_4$ | $R_6$ | n | A | p | physical data |
|---|---|---|---|---|---|---|---|---|---|
| 1 | $CH_3$ | $CH_3$ | H | H | 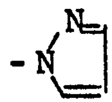 | 2 | Cl | - | mp.175-177° |
| 2 | $CH_3$ | $CH_3$ | H | H | 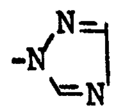 | 2 | Cl | - | mp.193-194° |
| 3 | $CH_3$ | $CH_3$ | $3-CH_3$ | H | 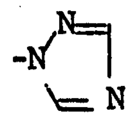 | 2 | Cl | - | mp.199-205° |
| 4 | $CH_3$ | $CH_3$ | $3-CH_3$ | $5-CH_3$ | 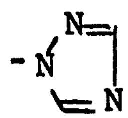 | 2 | Cl | - | |
| 5 | $CH_3$ | $CH_3$ | H | H | 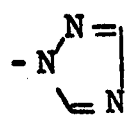 | 1 | $SO_4$ | - | |
| 6 | $CH_3$ | Cl | H | H | 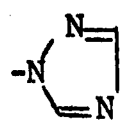 | 2 | AcO | - | |

| Cmpd. No. | $R_1$ | $R_2$ | $R_3$ | $R_4$ | $R_6$ | n | A | p | physical data |
|---|---|---|---|---|---|---|---|---|---|
| 7 | $CH_3$ | $CH_3$ | H | H | 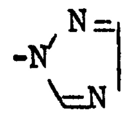 | 2 | $NO_3$ | - | |
| 8 | $CH_3$ | $CH_3$ | 3-Cl | H | 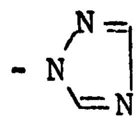 | 2 | Cl | - | |
| 9 | $CH_3$ | $CH_3$ | H | H | 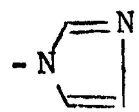 | 2 | Cl | - | |

Table II ($R_5$ = $CH(CH_3)CH_2OCH_3$)

| Cmpd. No. | $R_1$ | $R_2$ | $R_3$ | $R_4$ | $R_6$ | n | A | p | physical data |
|---|---|---|---|---|---|---|---|---|---|
| 10 | $CH_3$ | $CH_3$ | H | H | 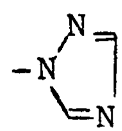 | 2 | Cl | - | mp.138-[illegible] |
| 11 | $CH_3$ | Cl | H | H | 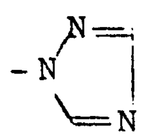 | 2 | Cl | - | mp.[illegible]- |

| Cmpd. No. | $R_1$ | $R_2$ | $R_3$ | $R_4$ | $R_6$ | n | A | p | physical data |
|---|---|---|---|---|---|---|---|---|---|
| 12 | $CH_3$ | $CH_3$ | 3-$CH_3$ | 5-$CH_3$ | 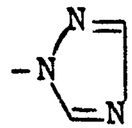 | 2 | Cl | - | [illegible] |
| 13 | $CH_3$ | $C_2H_5$ | H | H | 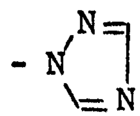 | 2 | Cl | - | mp.[illegible] |
| 14 | $CH_3$ | $CH_3$ | 3-Cl | H | 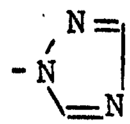 | 2 | Cl | - | |
| 15 | $CH_3$ | $CH_3$ | 4-Br | H | 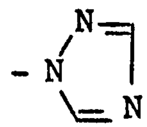 | 2 | Cl | - | |

Table III ($R_5$ = 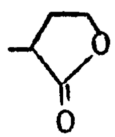)

| Cmpd. No. | $R_1$ | $R_2$ | $R_3$ | $R_4$ | $R_6$ | n | A | p | physical data |
|---|---|---|---|---|---|---|---|---|---|
| 16 | $CH_3$ | $CH_3$ | H | H | 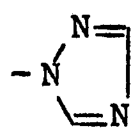 | 2 | Cl | - | mp.190-195° |

| Cmpd. No. | $R_1$ | $R_2$ | $R_3$ | $R_4$ | $R_6$ | n | A | p | physical data |
|---|---|---|---|---|---|---|---|---|---|
| 17 | $CH_3$ | $CH_3$ | [illegible] | " | 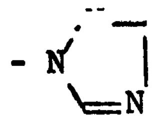 | 2 | Cl | - | |
| 18 | $CH_3$ | $CH_3$ | 3-$CH_3$ | 5-$CH_3$ | 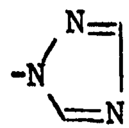 | 2 | Cl | - | |
| 19 | $CH_3$ | $CH_3$ | H | H | 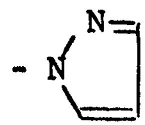 | 2 | Cl | - | |
| 20 | $CH_3$ | $CH_3$ | 3-Cl | H | 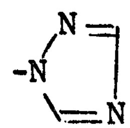 | 2 | Cl | - | |
| 21 | $CH_3$ | $CH_3$ | 4-Cl | H | 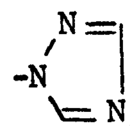 | 2 | Cl | - | |

The following are examples of compounds of the formula II. which can be used in forming complexes

Table IV

($R_5$ = $-\overset{CH_3}{\overset{|}{C}H}COOCH_3$; $R_6$ = 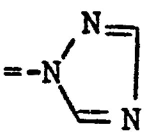 )

| $R_1$ | $R_2$ | $R_3$ | $R_4$ | physical data |
|---|---|---|---|---|
| $CH_3$ | $CH_3$ | H | H | mp.131-132° |
| $CH_3$ | $CH_3$ | 3-$CH_3$ | H | Harz |
| $CH_3$ | $C_2H_5$ | H | H | bp.162°/0.01 |
| $CH_3$ | $CH_3$ | 4-Cl | H | mp.135-137° |
| $CH_3$ | $C_2H_5$ | 4-Br | H | mp. 119-120° |
| $CH_3$ | H | 5-$CH_3$ | H | mp. 130-132° |
| $CH_3$ | Cl | H | H | mp. 109-112° |
| $CH_3O$ | $CH_3$ | H | H | mp.150-152° |
| $CH_3$ | $CH_3$ | 3-$CH_3$ | 5-$CH_3$ | mp.105-108° |
| $CH_3$ | i-$C_3H_7$ | H | H | mp. 95-98° |
| $CH_3$ | $CH_3$ | 4-$CH_3$ | H | mp. 118-119° |
| $CH_3$ | $CH_3$ | 4-Br | H | mp. 146° |

Table V

($R_5$ = $-\overset{CH_3}{CH}COOCH_3$; $R_6$ = 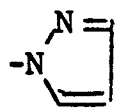 )

| $R_1$ | $R_2$ | $R_3$ | $R_4$ | physical data |
|---|---|---|---|---|
| $CH_3$ | $CH_3$ | H | H | mp. 102-104° |
| $CH_3$ | $CH_3$ | 3-$CH_3$ | H | viscous |
| $CH_3$ | $CH_3$ | 3-$CH_3$ | 5-$CH_3$ | bp.162°/0.001 |
| $CH_3$ | $C_2H_5$ | H | H | bp.108°/0.03 |
| $C_2H_5$ | $C_2H_5$ | H | H | bp.132°/0.12 |
| $CH_3$ | Cl | H | H | bp.115°/0.03 |
| $CH_3$ | Br | 4-Cl | H | bp.153°/0.011 |
| $CH_3O$ | $CH_3$ | H | H | bp.124°/0.08 |
| $CH_3O$ | Cl | H | H | bp.148°/0.04 |

Table VI

($R_5$ = 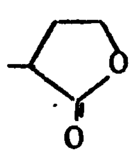 ; $R_6$ = 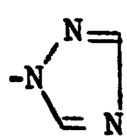 )

| $R_1$ | $R_2$ | $R_3$ | $R_4$ | physical data |
|---|---|---|---|---|
| $CH_3$ | $CH_3$ | H | H | mp.133-135° |
| $CH_3$ | Cl | H | H | |
| $CH_3$ | $C_2H_5$ | H | H | |
| $CH_3$ | Cl | H | H | |

| $R_1$ | $R_2$ | $R_3$ | $R_4$ | physical data |
|---|---|---|---|---|
| $CH_3$ | $CH_3$ | $3-CH_3$ | $5-CH_3$ | |
| $CH_3$ | $C_2H_5$ | H | H | |
| $CH_3$ | $CH_3$ | H | H | |
| $CH_3$ | $CH_3$ | $3-CH_3$ | $5-CH_3$ | |
| $CH_3$ | $CH_3$ | 3-Cl | H | |
| $CH_3$ | $CH_3$ | 3-Br | H | |
| $CH_3$ | $CH_3$ | $3-CH_3$ | H | decomp. at 70° |
| $CH_3$ | $CH_3$ | 3-Cl | H | |
| $CH_3$ | $CH_3$ | 3-Br | H | |
| $CH_3$ | $CH_3$ | 4-Cl | H | |
| $CH_3$ | $CH_3$ | $3-CH_3$ | H | |
| $CH_3$ | $CH_3$ | 4-Cl | H | |
| $CH_3$ | Br | 4-Cl | H | |
| $CH_3O$ | $CH_3$ | H | H | |
| $CH_3O$ | Cl | H | H | |

Table VII

($R_5$ = 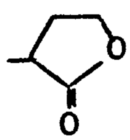 ; $R_6$ 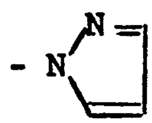 )

| $R_1$ | $R_2$ | $R_3$ | $R_4$ | physical data |
|---|---|---|---|---|
| $CH_3$ | $CH_3$ | H | H | |
| $CH_3$ | Cl | H | H | |
| $CH_3$ | $C_2H_5$ | H | H | |
| $CH_3$ | Cl | H | H | |
| $CH_3$ | $CH_3$ | $3-CH_3$ | $5-CH_3$ | |
| $CH_3$ | $C_2H_5$ | H | H | |
| $CH_3$ | $CH_3$ | H | H | |
| $CH_3$ | $CH_3$ | $3-CH_3$ | $5-CH_3$ | |
| $CH_3$ | $CH_3$ | 3-Cl | H | |

| $R_1$ | $R_2$ | $R_3$ | $R_4$ | physical data |
|---|---|---|---|---|
| $CH_3$ | $CH_3$ | 3-Br | H | |
| $CH_3$ | $CH_3$ | 3-$CH_3$ | H | mp.167-9° |
| $CH_3$ | $CH_3$ | 3-Cl | H | |
| $CH_3$ | $CH_3$ | 3-Br | H | |
| $CH_3$ | $CH_3$ | 4-Cl | H | |
| $CH_3$ | $CH_3$ | 3-$CH_3$ | H | |
| $CH_3$ | $CH_3$ | 4-Cl | H | |
| $CH_3$ | Br | 4-Cl | H | |
| $CH_3O$ | $CH_3$ | H | H | |
| $CH_3O$ | Cl | H | H | |

Table VIII ($R_5$ = $\overset{CH_3}{\overset{|}{C}}HCH_2OCH_3$ ; $R_6$ = 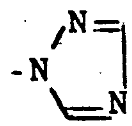 )

| $R_1$ | $R_2$ | $R_3$ | $R_4$ | physical data |
|---|---|---|---|---|
| $CH_3$ | $CH_3$ | H | H | mp. 91-93° |
| $CH_3$ | $CH_3$ | 3-$CH_3$ | H | bp.180-185° /0.05 Torr |
| $CH_3$ | $C_2H_5$ | H | H | oil |
| $CH_3$ | $CH_3$ | 4-Cl | H | oil |
| $CH_3$ | $CH_3$ | 3-Cl | H | oil |
| $CH_3$ | H | 5-$CH_3$ | H | viscous |
| $CH_3$ | Cl | H | H | mp. 95-102° |
| $CH_3O$ | $CH_3$ | H | H | oil |
| $CH_3$ | $CH_3$ | 3-$CH_3$ | 5-$CH_3$ | oil |
| $CH_3$ | iso$C_3H_7$ | H | H | oil |
| $CH_3$ | $CH_3$ | 4-sec$C_4H_9O$ | H | viscous oil |
| $CH_3$ | Cl | 4-Br | H | oil |

Table IX (R$_5$ = $\overset{CH_3}{\overset{|}{C}}HCH_2OCH_3$ ; R$_6$ = 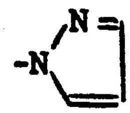 )

| $R_1$ | $R_2$ | $R_3$ | $R_4$ | physical data |
|---|---|---|---|---|
| $CH_3$ | $CH_3$ | H | H | dark oil |
| $CH_3$ | $CH_3$ | 3-$CH_3$ | H | oil |
| $CH_3$ | $CH_3$ | 3-$CH_3$ | 5-$CH_3$ | oil |
| $CH_3$ | $C_2H_5$ | H | H | |
| $CH_3$ | $CH_3$ | 4-$OC_2H_5$ | H | |
| $CH_3$ | Cl | H | H | |
| $CH_3$ | Cl | 4-Cl | H | |
| $CH_3O$ | $CH_3$ | H | H | |
| $CH_3O$ | Cl | H | H | |
| $CH_3$ | $CH_3$ | 4-Cl | H | oil |

Table X (R$_5$ = $\overset{CH_3}{\overset{|}{C}}HCOOCH_3$ ; R$_6$ = 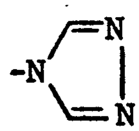 )

| $R_1$ | $R_2$ | $R_3$ | $R_4$ | physical data |
|---|---|---|---|---|
| $CH_3$ | $CH_3$ | H | H | |
| $CH_3$ | $CH_3$ | 3-$CH_3$ | H | |
| $CH_3$ | Cl | H | H | |
| $CH_3$ | $CH_3$ | 3-$CH_3$ | 5-$CH_3$ | |
| $CH_3$ | $C_2H_5$ | H | H | |

Table XI ($R_5$ = $CH(CH_3)CH_2OCH_3$ ; $R_6$ = ·

| $R_1$ | $R_2$ | $R_3$ | $R_4$ |
|---|---|---|---|
| $CH_3$ | $CH_3$ | H | H |
| $CH_3$ | $CH_3$ | 3-$CH_3$ | H |
| $CH_3$ | $CH_3$ | 3-$CH_3$ | 5-$CH_3$ |
| $CH_3$ | $CH_3$ | 4-$CH_3$ | H |
| $CH_3$ | $CH_3$ | 4-Cl | H |
| $CH_3$ | $CH_3$ | 4-Br | H |
| $CH_3$ | $C_2H_5$ | 4-Cl | H |
| $CH_3O$ | Cl | 4-Cl | H |
| $C_2H_5$ | H | 4-sec$C_4H_9O$ | H |
| $CH_3$ | $CH_3$ | 4-sec.$C_4H_9O$ | H |
| $CH_3$ | $CH_3$ | 3-Br | H |
| $CH_3$ | $CH_3$ | 3-Cl | H |

B. Formulation examples

Example 4

Dusts: The following substances are used to prepare

a) 5 % and b) a 2 % dust:

a) 5 parts of active substance
95 parts of talc;

b) 2 parts of active substance
1 part of highly dispersed silicic acid
97 parts of talc.

The active substances are mixed with the carriers and ground and in this form can be processed to dusts for application.

Example 5

Granulate. The following substances are used to prepare a 5 % granulate:

| | |
|---|---|
| 5 | parts of active substances |
| 0.25 | part of epichlorohydrin |
| 0.25 | part of cetyl polyglycol ether |
| 3.50 | parts of polyethylene glycol |
| 91 | parts of kaolin (particle size 0.3 - 0.8 mm). |

The active substance is mixed with epichlorohydrin and the mixture is dissolved in 6 parts of acetone. Then polyethylene glycol and cetyl polyglycol ether are added. The resultant solution is sprayed on kaolin and the acetone is evaporated in vacuo. Such a microgranulate may be used for example in combating soil fungi.

Example 6

Wettable powders: The following constituents are used to prepare a) a 70 %, b) a 40 %, c) and d) a 25 % and e) a 10 % wettable powder:

a) 70 parts of active substance
5 parts of sodium dibutylnaphthylsulphonate
3 parts of naphthalenesulphonic acid/phenolsulphonic acid/formaldehyde condensate (3:2:1)
10 parts of kaolin
12 parts of Champagne chalk

b) 40 parts of active substance
5 parts of sodium ligninsulphonate
1 part of sodium dibutylnaphthalenesulphonic acid
54 parts of silicic acid

c) 25 parts of active substance
   4.5 parts of calcium ligninsulphonate
   1.9 parts of Champagne chalk/hydroxyethyl cellulose mixture (1:1)
   1.5 parts of sodium dibutylnaphthalenesulphonate
   19.5 parts of silicic acid
   19.5 parts of Champagne chalk
   28.1 parts of kaolin

d) 25 parts of active substance
   2.5 parts of isooctylphenoxy-polyethylene-ethanol
   1.7 parts of a Champagne chalk/hydroxyethyl cellulose mixture (1:1)
   8.3 parts of sodium aluminium silicate
   16.5 parts of kieselguhr
   46 parts of kaolin

e) 10 parts of active substance
   3 parts of a mixture of the sodium salts of saturated fatty alcohol sulphates
   5 parts of naphthalenesulphonic acid/formaldehyde condensate
   82 parts of kaolin.

The active substances are intimately mixed in suitable mixers with the additives and ground in appropriate mills and rollers. Wettable powders of excellent wettability and suspension power are obtained. These wettable powders can be diluted with water to give suspensions of the desired concentration and can be used in particular for leaf application.

Example 7

Emulsifiable concentrates: The following substances are used to prepare a 25 % emulsifiable concentrate:

25 parts of active substance
2.5 parts of epoxidised vegetable oil
10 parts of an alkylarylsulphonate/fatty alcohol polyglycol ether mixture
5 parts of dimethyl formamide
57.5 parts of xylene.

By diluting such a concentrate with water it is possible to prepare emulsions of the desired concentration, which are especially suitable for leaf application.

C. Biological Examples

Example 8

Action against Plasmopara viticola on vines

Three greenhouse reared vine seedlings of the variety "chasselas" in the 10 leaf stage were sprayed with a spray broth (active substance concentration 0,06 %) prepared from a wettable powder containing the active substance to be tested. 24 hours later the plants were infected on the underside of the leaves with a spore suspension of the fungus. The plants were then kept in a humid chamber for 8 days whereupon symtoms of disease became visible on the control plants. The effectiveness of the tested substances was assessed by determing the number and size of the infected areas on the treated plants in comparison with the untreated but infected control plants.

Example 9

Action against Cercospora arachidicola on ground nut plants

Three-week-old ground nut plants were sprayed with a spray broth (containing 0.02 % of active substance) prepared from a wettable powder of the active substance. After approximately 48 hours the treated plants were infected by dusting with a conidia suspension of the fungus The infected plants were incubated for 3 days at 90 % relative humidity and then placed in a greenhouse at approx. 22° C. The fungal attack was evaluated after 12 days.

Example 10

Action against Phytophthora infestans on tomato plants

a) Curative Action

"Roter Gnom" tomato plants were sprayed when 3 weeks old with a zoospore suspension of the fungus and incubated for 24 hours at 18° to 20° C and 100 % humidity. After drying, the plants were sprayed with a broth (active substance concentration 0,06 %) prepared from a wettable powder containing the active substance. After the spray coating had dried, the plants were returned to the humid chamber for a further 4 days. The effectiveness of the tested substances was assessed by determining the size and number of the typical leaf specks which had appeared on the treated plants in comparison with the untreated but infected control plants.

b) Preventive-systemic action

The active substance, formulated as a wettable powder, was applied in a concentration of 0.006 % (referred to the volume of the soil) to the surface of the soil in pots containing 3-week-old "Roter Gnom" tomato plants. Three days later the underside of the leaves of the plants was sprayed with a zoospore suspension of the fungus. The plants were then kept in a spray chamber at 18° to 20° C and 100 % humidity for 5 days whereupon typical leaf specks appeared.

Example 11

Action against Pythium debaryanum on sugar beets and maize

The fungus was cultivated on sterile oat grains and added to a mixture of earth and sand. Sugar beet and maize seeds were then sown into flower pots filled with the infected soil. Immediately after sowing, the active substance was poured in the form of aqueous suspension over the soil (20 ppm of active substance referred to the volume of the soil). The pots were then placed for 2-3 weeks in a greenhouse at 20°-24° C. The soil was kept uniformly moist by gently spraying it with water. The emergence of the plants as well as the relative number of healthy and sick plants were determined.

Results

The followings compounds, amongst others, were succesful in reducing the fungal attack to less than 20 % compared with control plants.

Plasmopara viticola: Cmpds. nos. 2 3. 10, 11 and 16

Cercospora arachidicola: Cmpd. no. 10

Phytophthora infestans: Cmpds. nos. 2, 3, 10, 11, 13 and 16

Pythium debaryanum: Cmpds. nos. 2, 3, 10, 11 and 16.

<u>We claim:</u>

1. A copper complex of the formula I

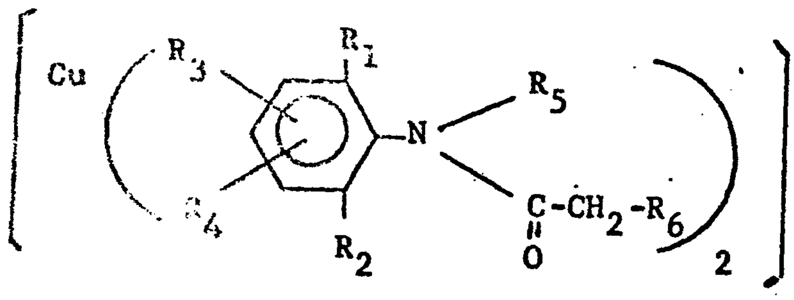 An . $pH_2O$ (I)

wherein

$R_1$ and $R_2$ represent independently of each other methyl, ethyl, methoxy, ethoxy or halogen,

$R_3$ represents hydrogen, $C_1$-$C_3$-alkyl, $C_1$-$C_4$-alkoxy or halogen,

$R_4$ represents hydrogen or methyl

$R_5$ represents $-\underset{CH_3}{CH}-COOCH_3$ ; $-\underset{CH_3}{CH}CH_2OCH_3$ or 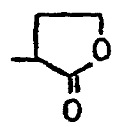

$R_6$ represents 1,2-pyrazole, 1,3-imidazole, 1,2,4-triazole (1) or 1,2,4-triazole (4)

A represents the anion of an organic or inorganic acid and

n stands for the number 1 or 2

p stands for a number from 1 to 12 inclusive.

2. A copper complex as claimed in claim 1 wherein

$R_1$ represents methyl,

$R_2$ represents methyl or chlorine and

$R_3$ represents hydrogen, chlorine, bromine or methyl.

3. A copper complex as claimed in claim 1 or 2 wherein $R_6$ represents the 1,2,4-triazole(1) group.

4. A copper complex as claimed in any one of claims 1 to 3 wherein

X represents the 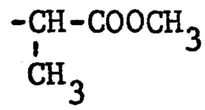 or 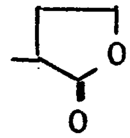 group

5. A copper complex as claimed in claim 4 wherein X represents the 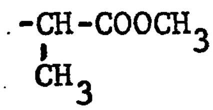 group.

6. A copper complex as claimed in any one of claims 1 to 5 wherein A represents the anion of an inorganic acid.

7. The copper complex of N-(1'-methoxycarbonyl-ethyl)-N-(1,2,4-triazol-1-yl)-acetyl-2,6-dimethylaniline of the formula

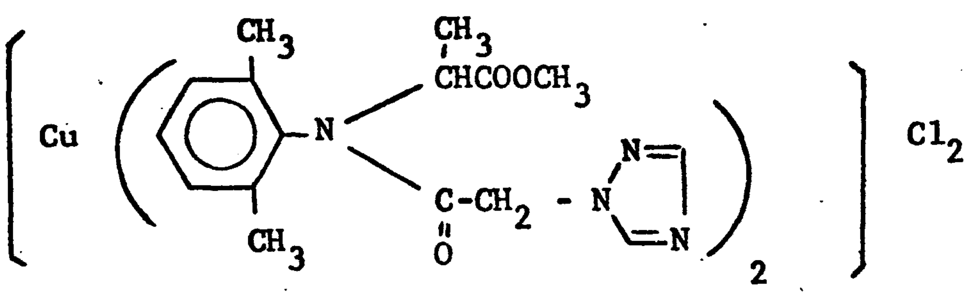

8. The copper complex of N-(1'-methoxycarbonyl-ethyl)-N-(1,2,4-triazol-1-yl)-acetyl-2,3,6-trimethylaniline of the formula

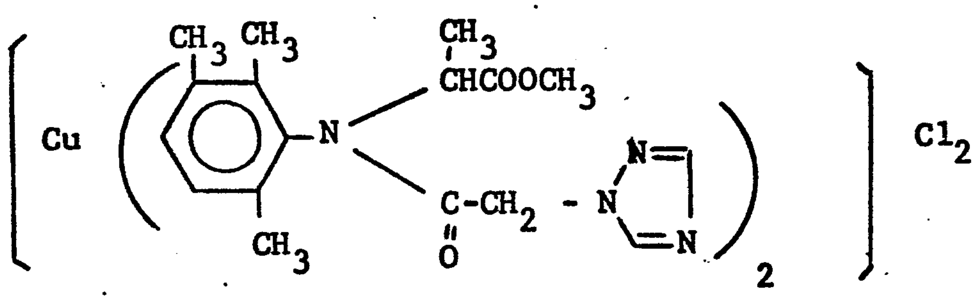

9. A process for preparing a copper complex of the formula I as defined in claim 1 characterised by reacting in the presence of an inert solvent a corresponding compound of the formula II

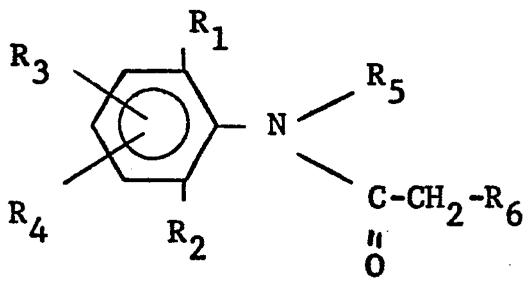

(II)

with a copper salt of the formula III

$$CuAn \cdot pH_2O \qquad (III)$$

whereby $R_1$ to $R_6$, A, n and p have the meanings given in claim 1.

10. A fungicidal composition containing as at least one active ingredient a copper complex as claimed in any one of claims 1 to 8.

11. A method for combatting phytopathogenic fungi which comprises applying thereto or to the locus thereof a copper complex as claimed in any one of claims 1 to 8 or a composition as claimed in claim 10.

0000539

European Patent Office

# EUROPEAN SEARCH REPORT

Application number
EP 78 10 043

## DOCUMENTS CONSIDERED TO BE RELEVANT

| Category | Citation of document with indication, where appropriate, of relevant passages | Relevant to claim |
|---|---|---|
| | NL - A - 76 10793 (CIBA-GEIGY)<br>* Claims; pages 28-32 *<br>-- | 1-11 |
| E | NL - A - 78 01231 (CIBA-GEIGY)<br>* Claims *<br>---- | 1-11 |

**CLASSIFICATION OF THE APPLICATION (Int. Cl.²)**

C 07 F 1/00
A 01 N 11/04

**TECHNICAL FIELDS SEARCHED (Int.Cl.²)**

C 07 D 249/08
C 07 D 233/60
C 07 D 231/12
A 01 N 9/22
C 07 F 1/00
A 01 N 11/04

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 06-11-1978 | CREMERS |

EPO Form 1503.1 06.78